# FASCICULE DE BREVET EUROPEEN

(11) **EP 0 685 487 B1**
(45) Date de publication et mention de la délivrance du brevet: **03.09.1997**
(21) Numéro de dépôt: 95401273.8
(22) Date de dépôt: 01.06.1995
(51) Int. Cl.: C07J 7/00, C07J 41/00, C07J 21/00, C07J 71/00

(54) **Nouveau procédé de préparation d'un stéroide 16bêta-méthyl et nouveaux intermédiaires**
Neues Verfahren zur Herstellung von einem 16-beta-steroid und neue Zwischenprodukte davon
A new method of preparation of a 16-beta-steroid and new intermediates of said preparation

(30) Priorité: 02.06.1994 FR 9406743
(43) Date de publication de la demande: 06.12.1995
(73) Titulaire: ROUSSEL-UCLAF, 93230 Romainville (FR)
(72) Inventeur: Geurts, Catherine, F-60620 Betz (FR); Vivat, Michel, F-77400 Lagny sur Marne (FR)
(74) Mandataire: Vieillefosse, Jean-Claude

(56) Documents cités:
- EP-A- 0 263 569
- GB-A- 2 199 325
- CHEMICAL ABSTRACTS, vol. 111, no. 9, 28 Août 1989 Columbus, Ohio, US; abstract no. 78494, Y. NI ET AL 'D-Homoannulation of 17-alpha-Hydroxy-16-beta-methyl-5,9(11)-pr egnadiene-3,20-dione-bis(ethylidene ketal)' page 796; colonne 1;
- TETRAHEDRON LETTERS., vol. 31, no. 24, 1990 OXFORD GB, pages 3475-3476, A. TOR ET AL 'Oxidative Decarboxylation of 17(20)-Dehydro-23,24-Dinorcholanoic Acids'
- CHEMICAL ABSTRACTS, vol. 85, no. 13, 27 Septembre 1976 Columbus, Ohio, US; abstract no. 94616, I. VILLAX ET AL 'Preparation of Steroids' page 662; colonne 2;
- CHEMICAL ABSTRACTS, vol. 101, no. 3, 16 Juillet 1984 Columbus, Ohio, US; abstract no. 023804, LI Z ET AL 'Studies on the epoxidation of 3.beta.-hydroxy-5,16-pregnadien-20-one acetate' page 631; colonne 1;

## Description

La présente invention a pour objet un procédé de préparation d'un stéroïde 16β-méthyl et des intermédiaires.

L'invention a ainsi pour objet un procédé de préparation du composé de formule (I) : 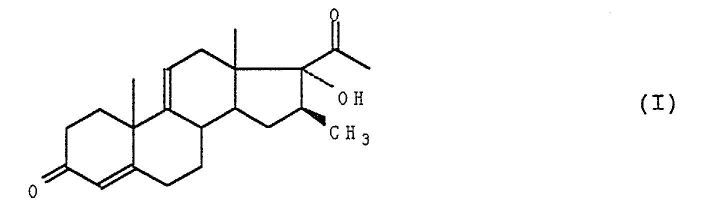 caractérisé en ce que l'on traite un composé de formule (II) : 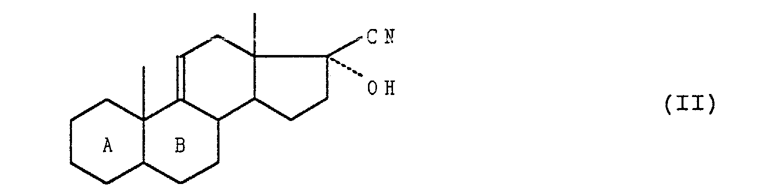 dans laquelle les cycles A et B représentent un reste : 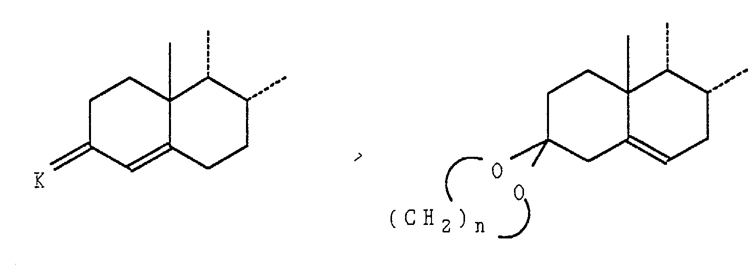 ou 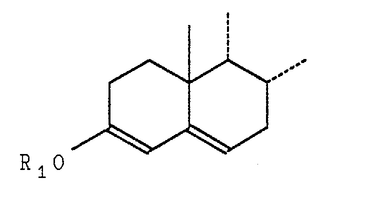 dans lequel K représente un radical oxo ou un groupement protecteur du radical oxo de formule : 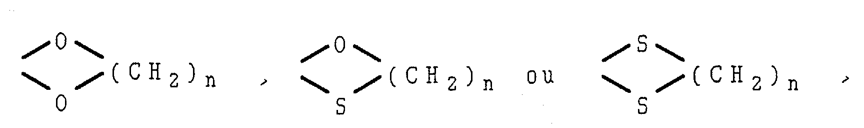 n est égal à 2 ou 3 et R₁ représente un reste éther ou ester, par un agent de déshydratation, pour obtenir un composé de formule (III) : 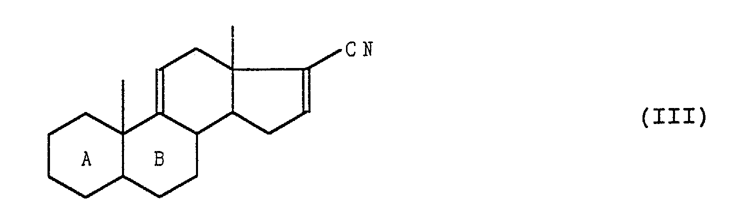 dans laquelle A et B ont la signification déjà indiquée, que lorsque K représente un radical oxo, l'on soumet à l'action d'un réactif de blocage approprié dudit radical oxo, pour obtenir le composé de formule (III) correspondant, dans laquelle K a les valeurs différentes du radical oxo, telles que définies précédemment, composé de formule (III) que l'on soumet à l'action d'un réactif de méthylation organométallique, pour obtenir après hydrolyse de l'imine intermédiaire, une méthyl cétone de formule (IV) : 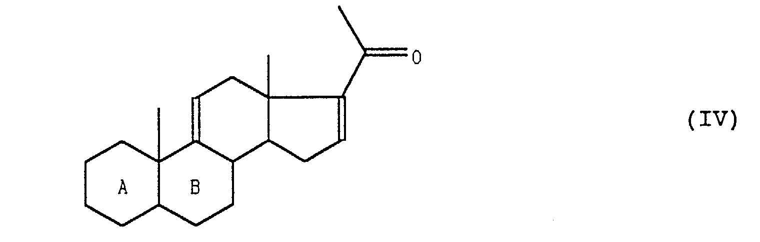 que l'on traite en milieu basique par un agent d'époxydation pour obtenir un composé de formule (V) : 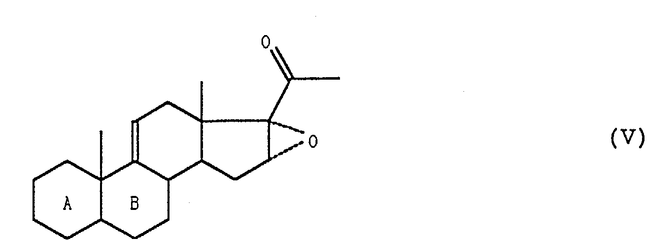 dont on protège la fonction 20-céto, pour obtenir un composé de formule (VI) : 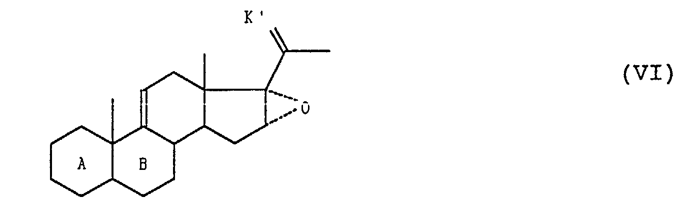 dans laquelle K' représente un groupement protecteur de la fonction cétone identique ou différent de K, de formule : 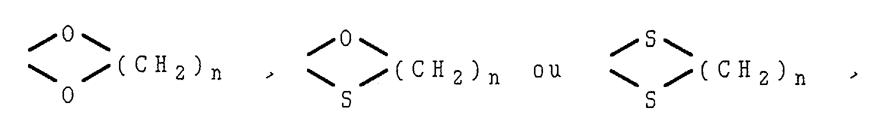 n étant défini comme précédemment, que l'on traite par un réactif de méthylation organométallique, pour obtenir le dérivé 16β-méthyl de formule (VII) : 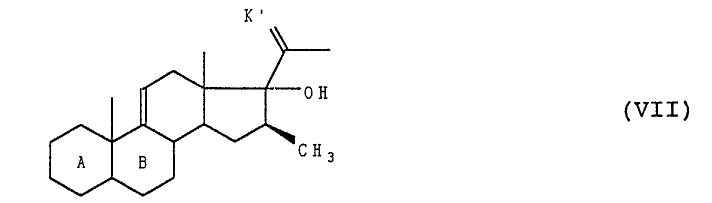 dont on débloque les fonctions cétone en 3 et 20, pour obtenir le produit de formule (I) attendu.

Lorsque R₁ représente un reste éther, il peut s'agir de tout reste connu de l'homme du métier pour bloquer la position 3 sous cette forme et notamment il peut s'agir d'un radical alkyle renfermant de 1 à 6 atomes de carbone, d'un radical alkoxyalkoxyalkyle renfermant de 3 à 8 atomes de carbone, d'un radical aryle renfermant de 6 à 10 atomes de carbone ou d'un radical aralkyle renfermant de 7 à 12 atomes de carbone.

Lorsque R₁ représente un radical alkyle, il s'agit par exemple d'un radical méthyle, éthyle, propyle, isopropyle, n-butyle, sec-butyle, tert-butyle, pentyle ou hexyle.

Lorsque R₁ représente un radical alkoxyalkoxyalkyle, il s'agit par exemple d'un radical méthoxyéthoxyméthyle.

Lorsque R₁ représente un radical aralkyle, il s'agit par exemple d'un radical benzyle ou phénéthyle.

Lorsque R₁ représente un radical aryle, il s'agit par exemple d'un radical phényle ou d'un radical phényle substitué, en particulier par un ou plusieurs radicaux alkyles.

Lorsque R₁ représente un reste éther, il peut encore s'agir d'un groupement silylé, par exemple d'un groupement trialkylsilyle tel que triméthylsilyle, tert-butyl diméthylsile ou encore par exemple d'un groupement triarylsilyle tel que triphénylsilyle ou diarylalkylsilyle tel que diphényl tert-butylsilyle.

Lorsque R₁ représente un reste ester, il peut s'agir de tout reste connu de l'homme du métier pour bloquer la position 3 sous cette forme et notamment il peut s'agir d'un reste -COR₁, R₁ étant un radical alkyle, aryle ou aralkyle tel que défini ci-dessus.

La protection de la fonction cétone en 3 est effectuée par des méthodes connues de l'homme du métier. On peut ainsi utiliser notamment un diol, un dithiol ou un thiol mixte de formule HO-(CH₂)ₙ-OH, HS-(CH₂)ₙ-SH ou HO-(CH₂)ₙ-SH, en milieu acide, par exemple en présence d'acide chlorhydrique ou bromhydrique concentré, en quantité catalytique, d'acide P-toluène sulfonique, ou encore en présence d'un acide de Lewis tel que le chlorure de zinc, le tétrachlorure de titane ou le trifluorure de bore de préférence sous forme d'éthérate. On peut encore utiliser le méthyléthyl dioxolane en présence d'un acide, par exemple l'un de ceux nommés ci-dessus.

On peut encore utiliser un halogénure d'alkyle, d'alkoxyalkoxyalkyle, d'aralkyle ou d'aryle, en présence d'une base formant intermédiairement l'énolate, par exemple un hydrure, un alcoolate ou un hydroxyde alcalin.

On peut encore utiliser un halogénure de trialkyl, triaryl ou diarylalkylsilyle en milieu alcalin comme ci-dessus.

On peut encore utiliser un chlorure d'acide approprié, en opérant en présence d'une base, qui peut être une base azotée, par exemple la triéthylamine, la pyridine, la diméthylaminopyridine, ou une base minérale, notamment un hydrure, un alcoolate ou un hydroxyde alcalin.

L'agent de déshydratation est notamment l'oxychlorure de phosphore, utilisé de préférence en présence d'une amine tertiaire telle que la pyridine. On peut encore utiliser un acide de Lewis tel que le chlorure ferrique, le trifluorure de bore et ses complexes, par exemple l'étherate, le tétrachlorure de titane, le chlorure d'aluminium ou encore le chlorure d'étain, un acide minéral tel que l'acide sulfurique, un acide sulfonique tel que l'acide paratoluène sulfonique ou l'acide chlorosulfonique ou un dérivé tel que le chlorure de méthane sulfonyle.

Le réactif de méthylation organo métallique conduisant à la méthylcétone est par exemple un magnésien, un lithien, un cadmien ou encore un dérivé du cuivre tel que CH₃Cu, (CH₃)₂CuMg ou (CH₃)₂CuLi. Un magnésien tel qu'un halogénure de méthylmagnésium ou le méthyllithium est plus particulièrement préféré.

On opère au sein d'un solvant qui est de préférence un éther tel que l'éther éthylique, le tétrahydrofuranne ou le dioxanne, mais qui peut aussi être un solvant aromatique tel que le toluène ou le xylène. On peut aussi opérer au sein d'un mélange éther-solvant aromatique.

L'hydrolyse de l'imine intermédiairement formée est réalisée par un acide aqueux, par exemple l'acide acétique, l'acide formique ou un acide minéral tel que l'acide chlorhydrique.

L'agent d'époxydation peut être un peracide tel que l'acide métachloroperbenzoïque, l'acide perphtalique, l'acide pertungstique ou encore l'eau oxygénée utilisée seule ou en présence d'hexachloro ou d'hexafluoroacétone.

L'agent d'époxydation peut encore être un hydroperoxyde tel que l'hydroperoxyde de tertbutyle, utilisé en présence d'acétyl acétonate de vanadium ou autres métaux tel que le molybdène, en quantité catalytique. L'eau oxygénée est plus particulièrement préférée.

On opère en milieu légèrement basique, soit en présence d'une base, par exemple la soude, soit en milieu tamponné, par exemple par l'acétate de sodium, le phosphate disodique ou le bicarbonate de sodium ou par le mélange phosphate trisodique-acide phosphorique.

On opère au sein d'un solvant organique tel que le chlorure de méthylène, le tétrachlorure de carbone, le chloroforme, le méthanol, le tétrahydrofuranne, le dioxanne, le toluène, l'acétate d'éthyle ou un mélange de ces solvants, le cas échéant en présence d'eau.

La protection de la fonction 20-céto est effectuée sous forme de cétal, cétal mixte ou dithiocétal, par des méthodes connues de l'homme du métier. On utilise ainsi un diol, dithiol ou un thiol mixte de formule HO-(CH₂)ₙ-OH, HS-(CH₂)ₙ-SH ou HO-(CH₂)ₙ-SH, en milieu acide, par exemple en présence d'acide chlorhydrique ou bromhydrique concentré, en quantité catalytique, d'acide p-toluène sulfonique, ou encore en présence d'un acide de Lewis tel que le chlorure de zinc, le tétrachlorure de titane ou le trifluorure de bore de préférence sous forme d'éthérate.

Le réactif de méthylation organométallique que l'on fait agir sur l'époxyde de formule (VI) est l'un de ceux qui ont été mentionnés plus haut, les réactifs préférés étant également ceux qui ont été mentionnés comme tels plus haut.

La libération des fonctions cétone en 3 et 20 est effectuée par des moyens appropriés à la nature du groupement protecteur. On utilise un agent acide en présence d'eau ou d'un mélange eau-alcanol, dans le cas d'un cétal. Il s'agit par exemple d'un acide minéral ou organique tel que l'acide chlorhydrique, bromhydrique, sulfurique, perchlorique, nitrique, paratoluène sulfonique, acétique, formique, oxalique ou d'un mélange d'acides, ou encore d'une résine acide, par exemple une résine sulfonique. Dans le cas d'un thiocétal ou d'un cétal mixte, la déprotection est réalisée par action de l'iode en présence d'une base, par exemple un bicarbonate alcalin, ou par action de l'iode en quantité catalytique, en présence d'une agent oxydant, notamment l'eau oxygénée, par action de l'iodure de méthyle, de l'acide glyoxylique, ou encore de sels de métaux, tels le mercure, le cadmium, le cuivre ou l'argent. On peut, en général, opérer dans un solvant tel qu'un alcanol inférieur, par exemple le méthanol ou l'éthanol, en mélange avec un solvant halogéné, par exemple le chlorure de méthylène, en présence d'eau. Dans le cas d'un cétal mixte, la déprotection est encore effectuée par exemple par un sel mercurique tel que le chlorure mercurique en présence d'un tampon acide acétique/acétate de potassium à 100°C environ, par le Nickel de Raney dans les mêmes conditions que ci-dessus ou par le mélange acide chlorhydrique-acide acétique à chaud.

Dans le cas où R₁ représente un reste éther ou ester, on utilise également un traitement acide, notamment dans les conditions décrites ci-dessus pour le cétal.

L'invention a notamment pour objet un procédé tel que défini précédemment, caractérisé en ce que l'on utilise au départ un composé de formule (II) dans laquelle les cycles A et B représentent un reste : 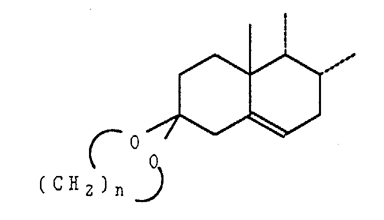 n étant défini comme précédemment et notamment égal à 2.

L'invention a aussi notamment pour objet un procédé tel que défini précédemment caractérisé en ce que l'on protège la fonction 20-céto par le même cétal qu'en position 3 et notamment par un groupement éthylènedioxy.

L'invention a encore pour objet, à titre de produits industriels nouveaux, et notamment à titre de produits intermédiaires utiles pour la mise en oeuvre du procédé de l'invention, le produit de formule (III), dans laquelle K a les définitions indiquées précédemment, à l'exception d'un radical oxo, ainsi que les produits de formules (IV), (V), (VI) et (VII) telles que définies précédemment.

Les produits de formule (II) utilisés au départ du procédé de l'invention sont connus ou peuvent être préparés au départ du produit 3-céto, décrit dans le brevet européen n° 263569, par des procédés connus de l'homme du métier. Parmi les références possibles, on peut citer le brevet français 1 079 781, le brevet est-allemand 281 394 ou la demande WO 88/03534.

Le produit de formule (I) est un intermédiaire important dans la synthèse de la bétamétasone ainsi que cela est décrit par exemple dans le brevet européen 54810.

L'exemple suivant illustre l'invention sans toutefois la limiter.

### EXEMPLE : 16β-méthyl 17α-hydroxy pregna 4,9(11)-diène 3,20-dione

### Stade A : 3-(1,2-éthanediyl) acétal cyclique de 17-cyano androst-5,9(11),16(17)-trièn-3-one

On mélange sous gaz inerte 30 g de 3-(1,2-éthanediyl) acétal cyclique de 17α-hydroxy 17β-cyano androst-5,9(11)dièn-3-one et 240 cm³ de pyridine anhydre puis ajoute 30 cm³ d'oxychlorure de phosphore. On maintient sous agitation pendant 2 h dans un bain d'huile à 40°C puis pendant 18 h à 45°C. On verse le mélange sur un mélange de 450 g de glace, 240 cm³ d'acide chlorhydrique à 22°Bé et 100 cm³ d'eau. On agite à température ambiante puis essore et lave les cristaux à l'eau puis les sèche. On met le produit brut en suspension dans un mélange éther isopropylique-isopropanol (2-1) puis essore et sèche les cristaux. On obtient 24,27 g de produit attendu. F = 205-208°C.
Spectre IR : (CHCl₃)
Absorptions à 2217 cm⁻¹ (CN), 1660, 1630 et 1593 cm⁻¹ (C=C).
Spectre RMN : (CDCl₃+ C₅D₅N - 300 MHz - ppm)
0,91 (s) : 18-CH₃ ; 1,23 (s) : 19-CH₃ ; 3,96 : cétal ; 5,44 (m)-5,45 (m) : H₆ et H₁₁ ; 6,65 : H₁₆.

### Stade B : 3-(1,2-éthanediyl) acétal cyclique de pregna 5,9(11),16(17)-triène-3,20-dione

On mélange sous gaz inerte 9 g de produit obtenu au stade A et 27 cm³ de toluène, puis introduit 27 cm³ d'une solution 3M de chlorure de méthylmagnésium dans le tétrahydrofuranne. On porte pendant 3 h 30 à 60/65°C puis refroidit par un bain glace-méthanol et ajoute 36 cm³ de tétrahydrofuranne, puis verse sous pression d'azote, à 10°C maximum sur le mélange de 81 cm³ de glace, 27 cm³ d'eau et 135 cm³ d'acide acétique refroidi par un bain glace-méthanol. On laisse remonter la température à 20°C environ, concentre sous pression réduite en chauffant légèrement par un bain marie à 45°C puis verse dans 20 volumes d'un mélange d'eau et de glace. On laisse remonter la température, sous agitation, puis essore les cristaux, les lave à l'eau et les sèche. On obtient 9,03 g de produit brut que l'on empâte dans le mélange de 45 cm³ de méthanol, 5 cm³ d'eau et 0,5 cm³ de triéthylamine. On chauffe au reflux pendant 1 h 30 puis refroidit à température ambiante, essore les cristaux, les lave au méthanol à 10 % d'eau et les sèche. On obtient 7,12 g de produit attendu. F = 217-219°C.
Spectre IR : (CHCl₃)
Absorptions à 1664, 1590, 1361 cm⁻¹ : méthyl cétone α,β insaturée.
Spectre RMN : (CDCl₃+ C₅D₅N - 300 MHz - ppm)
0,87 (s) : 18-CH₃ ; 1,22 (s) : 19-CH₃ ; 2,26 (s) : méthylcétone ; 3,94 (m) : cétal ; 5,44-5,45 (m) : -CH= en 11 et 5 ; 6,71 (t) : -CH= en 16.

### Stade C : 3-(1,2-éthanediyl) acétal cyclique de 16α,17α-époxy pregna 5,9(11)-diène-3,20-dione

On mélange sous gaz inerte 7 g de produit obtenu au stade B, 70 cm³ de tétrahydrofuranne et 35 cm³ de méthanol. On introduit 7 cm³ de soude concentrée et 7 cm³ d'eau oxygénée à 200 volumes. On chauffe à 40/45°C pendant 17 heures puis refroidit à température ambiante et verse sur un mélange de 70 cm³ d'eau et de 70 g de glace. On maintient sous agitation en laissant remonter la température puis essore, lave à l'eau à fin de pouvoir oxydant et sèche. On obtient 7,08 g de produit attendu utilisé tel quel pour le stade suivant.
Spectre IR : (CHCl₃)
Absorptions à 1704 (C=O), 901-855 cm⁻¹ (époxyde).
Spectre RMN : (CDCl₃+ C₅D₅N - 300 MHz - ppm)
1,00 (s) : 18-CH₃ ; 1,20 (s) : 19-CH₃ ; 2,04 (s) : méthylcétone ; 3,74 (s) : -CH= en 16 ; 3,94 (m) : cétal ; 5,40 (m) 5,51 (t) : -CH= en 11 et 5.

### Stade D : 3,20 bis-(1,2-éthanediyl) acétal cyclique de 16α,17α-époxy pregna 5,9(11)-diène-3,20-dione

On mélange sous gaz inerte 4 g de produit obtenu au stade C, 40 cm³ de chlorure de méthylène, 40 cm³ d'éthylène glycol, 20 cm³ d'orthoformiate d'éthyle et 0,3 g d'acide paratoluène sulfonique dihydraté. Après 7 heures, on concentre sous pression réduite de 1 mbar à température ambiante puis maintient sous agitation pendant 30 mn. On verse ensuite sous 300 cm³ d'une solution aqueuse de bicarbonate de sodium à 10 % puis dégaze pendant 30 mn. On essore, lave à l'eau puis sèche les cristaux formés. On purifie le produit par chromatographie sur silice en éluant au mélange toluène-acétate d'éthyle (8-2) à 0,05 % de triéthylamine. On reprend le produit obtenu par du cyclohexane, essore et sèche sous pression réduite. On obtient 3,28 g de produit attendu.
F = 176-177°C.
Spectre IR : (CHCl₃)
Absence de C=O. Présence de cétal.
Spectre RMN : (CDCl₃+ C₅D₅N - 300 MHz - ppm)
0,94 (s) : 18-CH₃ ; 1,20 (s) : 19-CH₃ ; 1,42 (s) : CH₃- ; 3,41 (s) : H en 16 ; 3,85 à 4,05 : cétal ; 5,41 et 5,48 : H en 6 et H en 11.

### Stade E : 3,20 bis-(1,2-éthanediyl) acétal cyclique de 16β-méthyl 17α-hydroxy pregna 5,9(11)-diène-3,20-dione

On glace sous gaz inerte 24 cm³ d'une solution 3M de bromure de méthyl magnésium dans l'éther. On évapore le solvant sous pression réduite et en tiédissant par un bain à 60/65°C. On introduit ensuite 21 cm³ de tétrahydrofuranne et 3 g de produit obtenu au stade D, puis agite pendant 16 h en portant la température du bain à 70°C. On rajoute 4 cm³ de solution éthérée de magnésien ci-dessus et 10 cm³ de tétrahydrofuranne et agite pendant 7 h. On rajoute de nouveau 10 cm³ de tétrahydrofuranne, maintient au reflux pendant 16 h puis refroidit vers 40°C et verse sur un mélange maintenu à 0°C de 16 g de phosphate acide de sodium dihydraté et 900 cm³ d'eau. On laisse remonter la température puis essore et lave le produit à l'eau et le sèche. On purifie le produit par empâtage dans 15 volumes de mélange chlorure de méthylène-acétate d'éthyle (9-1) à 0,05 % de triéthylamine, puis par chromatographie sur silice en éluant au même mélange et obtient 2,42 g de produit attendu. F = 180-181°C.
Spectre IR : (CHCl₃)
Absorptions à 3580 cm⁻¹ : OH ; 1670-1644 cm⁻¹ : C=C.
Spectre RMN : (CDCl₃+ C₅D₅N - 300 MHz - ppm)
0,87 (s) : 18-CH₃ ; 1,19 (d) : CH₃-CH- ; 1,20 (s) : 19-CH₃ ; 1,39 (s) : CH₃-C- ; 3,85 à 4,05 : cétals ; 5,45 et 5,48 : H en 6 et H en 11.

### Stade F : 16β-méthyl 17α-hydroxy pregna 4,9(11)-diène 3,20-dione

On mélange sous gaz inerte 2 g de produit obtenu au stade E, 15 cm³ d'acétone et 5 cm³ d'eau. On introduit 4 gouttes d'acide sulfurique concentré et chauffe pendant 4 h 30 mn dans un bain à 60°C. On ajoute ensuite 20 cm³ d'eau puis laisse refroidir, essore les cristaux et les lave à l'eau puis les sèche. On obtient 1,55 g de produit attendu. F = 173°C.
Spectre IR : (CHCl₃)
Absorptions à 3610 cm⁻¹ : -OH ; 1709-1353 cm⁻¹ : CO-CH₃ ; 1663-1616 cm⁻¹ : céto 3 Δ 4.
Spectre RMN : (CDCl₃ - 300 MHz - ppm)
0,87 (s) : 18-CH₃ ; 1,19 (d,J = 7) : CH₃-CH- ; 1,34 (s) : 19-CH₃ ; 2,27 (s) : -CO-CH₃ ; 3,01 (s) : 1H mobile ; 5,52 (m) : H en 11 ; 5,75 (m) : H en 4.

## Revendications

1. Procédé de préparation du composé de formule (I) : 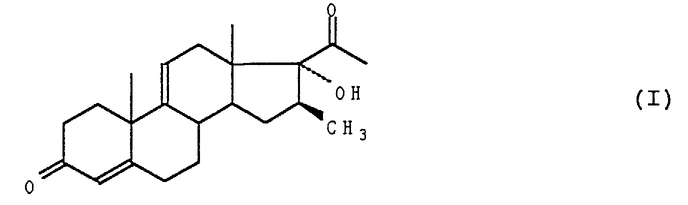 caractérisé en ce que l'on traite un composé de formule (II) : 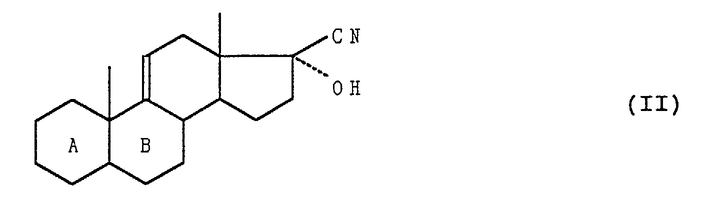 dans laquelle les cycles A et B représentent un reste : 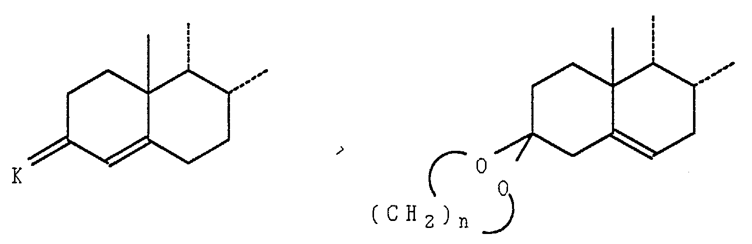 ou 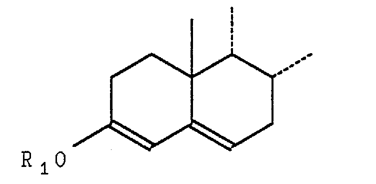 dans lequel K représente un radical oxo ou un groupement protecteur du radical oxo de formule : 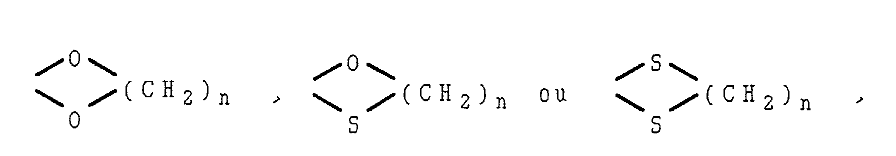 n est égal à 2 ou 3 et R₁ représente un reste éther ou ester, par un agent de déshydratation, pour obtenir un composé de formule (III) : 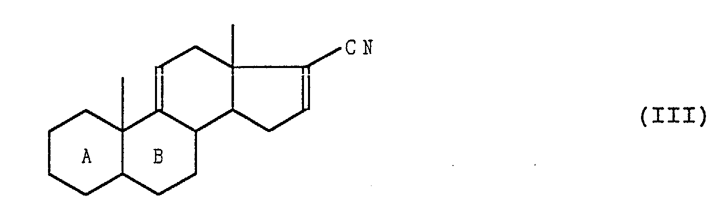 dans laquelle A et B ont la signification déjà indiquée, que, lorsque K représente un radical oxo, l'on soumet à l'action d'un réactif de blocage approprié dudit radical oxo, pour obtenir le composé de formule (III) correspondant, dans laquelle K a les valeurs différentes du radical oxo, telles que définies précédemment, composé de formule (III) que l'on soumet à l'action d'un réactif de méthylation organométallique, pour obtenir après hydrolyse de l'imine intermédiaire par un acide aqueux, une méthyl cétone de formule (IV) : 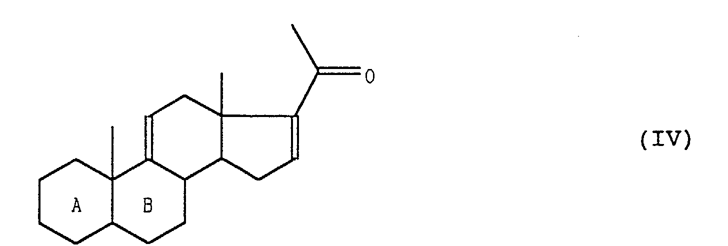 que l'on traite en milieu basique par un agent d'époxydation pour obtenir un composé de formule (V) : 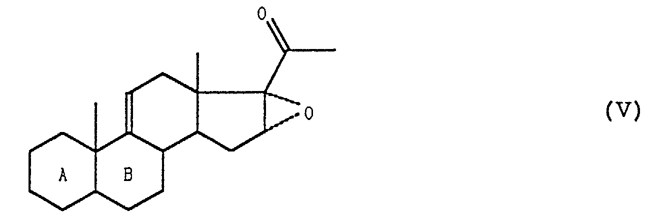 dont on protège la fonction 20-céto, pour obtenir un composé de formule (VI) **:** 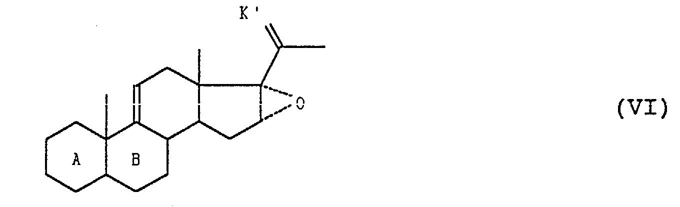 dans laquelle K' représente un groupement protecteur de la fonction cétone identique ou différent de K, de formule : 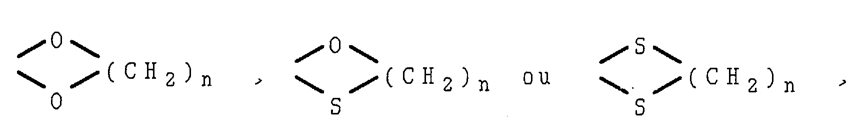 n étant défini comme précédemment, que l'on traite par un réactif de méthylation organométallique, pour obtenir le dérivé 16β-méthyl de formule (VII) : 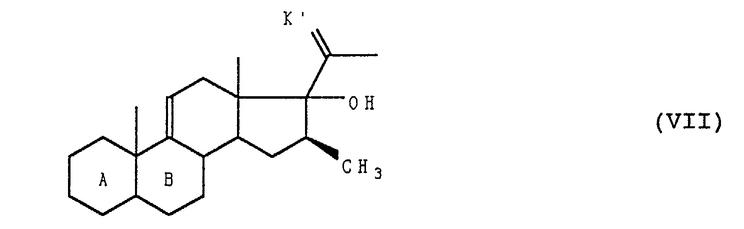 dont on débloque les fonctions cétone en 3 et 20, pour obtenir le produit de formule (I) attendu.

2. Procédé selon la revendication 1, caractérisé en ce que l'agent de déshydratation est l'oxychlorure de phosphore, utilisé en présence d'une amine tertiaire.

3. Procédé selon la revendication 1, caractérisé en ce que le réactif de méthylation organométallique est un halogénure de méthylmagnésium ou le méthyllithium.

4. Procédé selon la revendication 1, caractérisé en ce que le réactif d'époxydation est l'eau oxygénée.

5. Procédé selon la revendication 1, caractérisé en ce que le réactif de méthylation organométallique que l'on fait agir sur l'époxyde de formule (VI) est l'un de ceux mentionnés à la revendication 3.

6. Procédé selon l'une quelconque des revendications 1 à 5, caractérisé en ce que l'on utilise au départ un composé de formule (II) dans laquelle les cycles A et B représentent un reste : 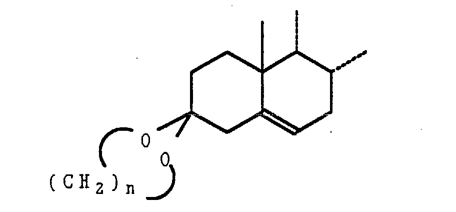 n étant défini comme précédemment et notamment égal à 2.

7. Procédé selon l'une quelconque des revendications 1 à 6, caractérisé en ce que l'on protège la fonction 20-céto par le même cétal qu'en position 3 et notamment parun groupement éthylènedioxy.

8. A titre de produits industriels, le composé de formule (III), telle que définie à la revendication 1, dans laquelle K a les valeurs définies à la revendication 1, à l'exception du radical oxo, ainsi que les composés de formules (IV), (V), (VI) et (VII) telles que définies à la revendication 1, à l'exclusion des composés 3,3-éthylèndioxy-5.9(11),16-pregnatirèn-20-one et 3,3; 20,20-bis(ethylèndioxy)-17α-hydroxy-16β-méthyle-5,9(11)-pregnadiène.

## Claims

1. Preparation process for the compound of formula (I): 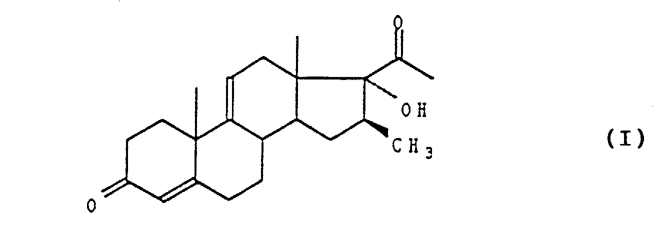 characterized in that a compound of formula (II): 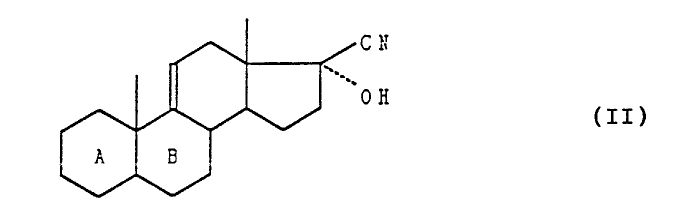 in which rings A and B represent a remainder: 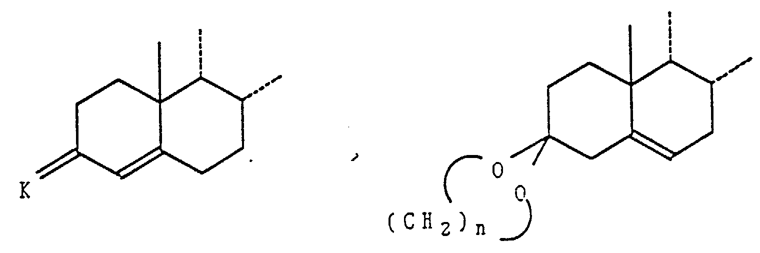 or 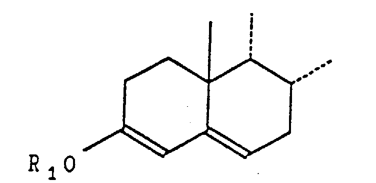 in which K represents an oxo radical or a protector group of the oxo radical of formula: 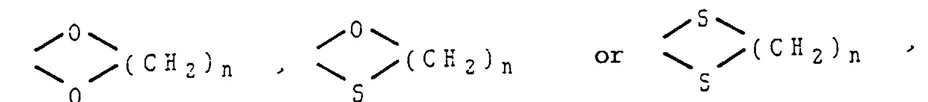 n is equal to 2 or 3 and R₁ represents an ether or ester remainder, is treated with a dehydration agent, in order to obtain a compound of formula (III): 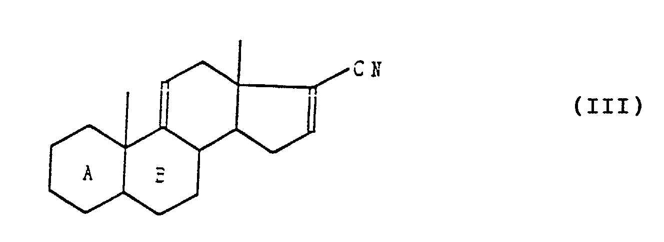 in which A and B have the meaning already indicated, which, when K represents an oxo radical, is subjected to the action of a suitable blocking agent of the oxo radical, in order to obtain the corresponding compound of formula (III), in which K has values different from the oxo radical, as defined previously, which compound of formula (III) is subjected to the action of an organometallic methylation agent, in order to obtain, after hydrolysis of the intermediate imine by an aqueous acid, a methyl ketone of formula (IV): 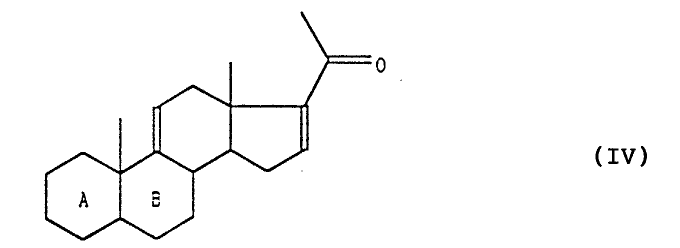 which is treated in a basic medium with an epoxidation agent in order to obtain a compound of formula (V): 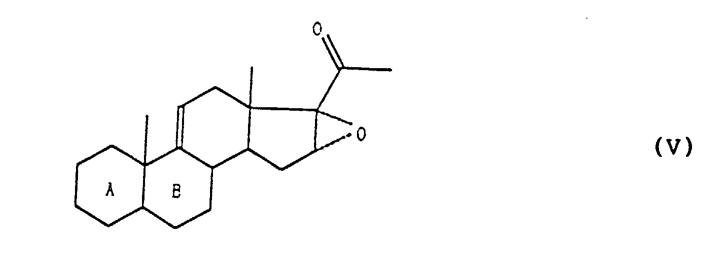 the 20-keto function of which is protected, in order to obtain a compound of formula (VI): 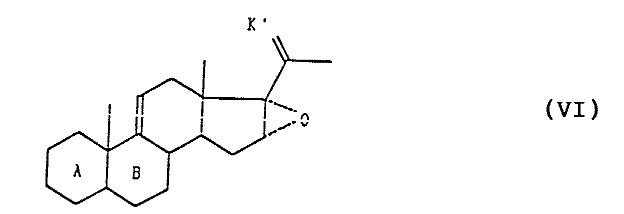 in which K' represents a protector group of the ketone function identical to or different from K, of formula: 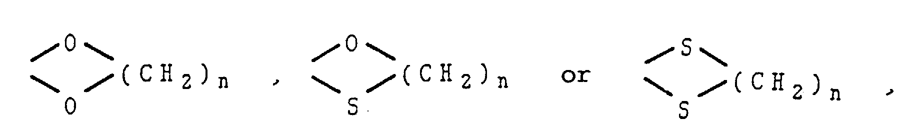 n being defined as previously, which is treated with an organometallic methylation agent, in order to obtain the 16 - methyl derivative of formula (VII): 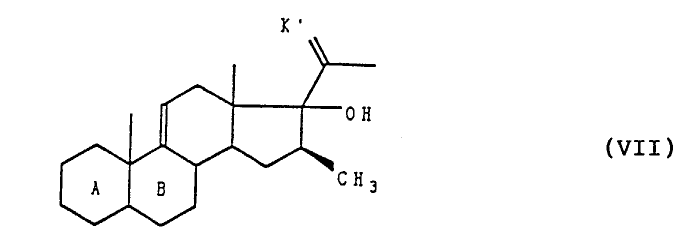 of which the ketone functions in positions 3 and 20 are unblocked, in order to obtain the expected product of formula (I).

2. Process according to claim 1, characterized in that the dehydration agent is phosphorus oxychloride, used in the presence of a tertiary amine.

3. Process according to claim 1, characterized in that the organometallic methylation agent is a methylmagnesium halide or methyllithium.

4. Process according to claim 1, characterized in that the epoxidation agent is hydrogen peroxide.

5. Process according to claim 1, characterized in that the organometallic methylation agent which is reacted with the epoxide of formula (VI) is one of those mentioned in claim 3.

6. Process according to any one of claims 1 to 5, characterized in that a compound of formula (II) is used at the start in which rings A and B represent a remainder: 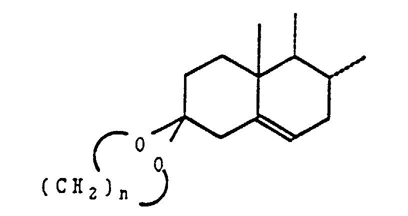 n being defined as previously and in particular equal to 2.

7. Process according to any one of claims 1 to 6, characterized in that the 20-keto function is protected by the same ketal as in position 3 and in particular by an ethylenedioxy group.

8. As industrial products, the compound of formula (III), as defined in claim 1, in which K has the values defined in claim 1, with the exception of the oxo radical, as well as the compounds of formulae (IV), (V), (VI) and (VII) as defined in claim 1, with the exclusion of the compounds 3,3-ethylenedioxy-5,9(11),16-pregnatrien-20-one and 3,3;20,20-bis(ethylenedioxy)-17alpha-hydroxy-16beta-methyl-5,9(11)-pregnadiene.

## Patentansprüche

1. Verfahren zur Herstellung der Verbindung der Formel (I): 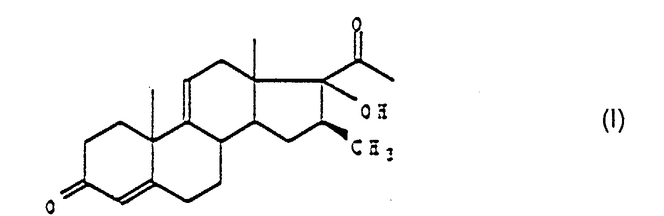 dadurch gekennzeichnet, daß man eine Verbindung der Formel (II): 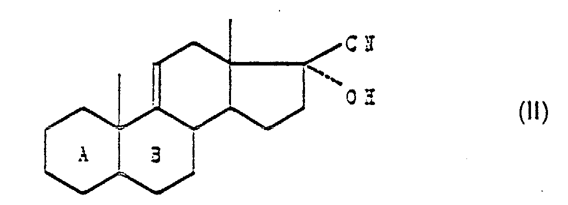 in welcher die Ringe A und B einen Rest: 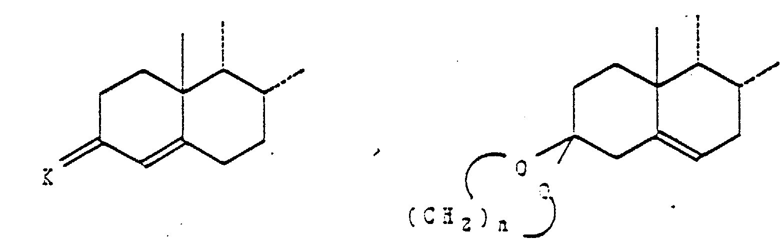 oder 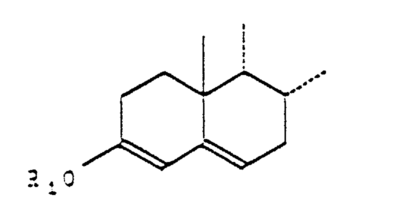 darstellen, in welchem K einen Oxorest oder eine Schutzgruppe für den Oxorest der Formel: 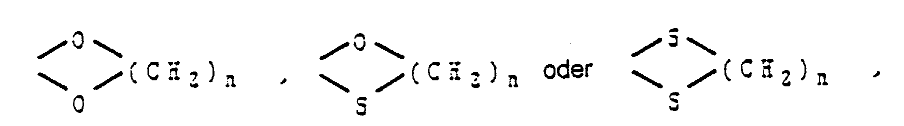 darstellt, n gleich 2 oder 3 ist und R₁ einen Ether- oder Esterrest darstellt, mit einem Dehydratisierungsmittel behandelt, um eine Verbindung der Formel (III): 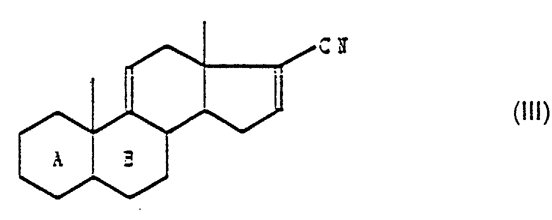 zu erhalten, in welcher A und B die bereits angegebene Bedeutung besitzen, die man, wenn K einen Oxorest darstellt, der Wirkung eines für besagten Oxorest geeigneten Blockierungsreagenzes unterwirft, um die entsprechende Verbindung der Formel (III) zu erhalten, in welcher K die wie vorhergehend definierten von dem Oxorest verschiedenen Werte besitzt; die Verbindung der Formel (III) unterwirft man der Wirkung eines organometallischen Methylierungsreagenzes, um nach Hydrolyse des intermediären Imins durch eine wäßrige Säure ein Methylketon der Formel (IV): 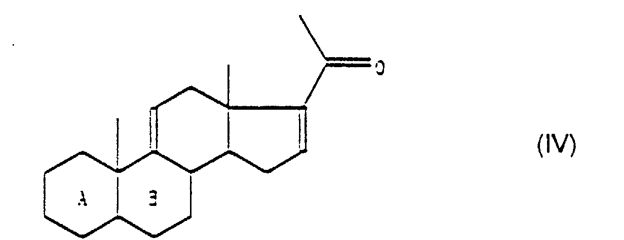 zu erhalten, das man in basischem Medium mit einem Epoxydierungsmittel behandelt, um eine Verbindung der Formel (V): 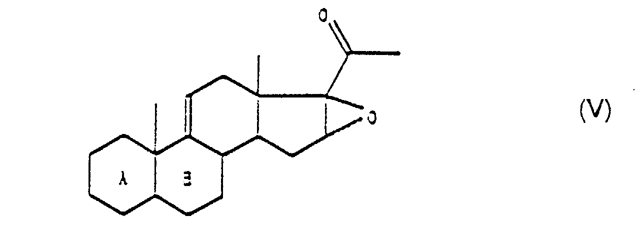 zu erhalten, deren 20-Keto-Funktion man schützt, um eine Verbindung der Formel (Vl): 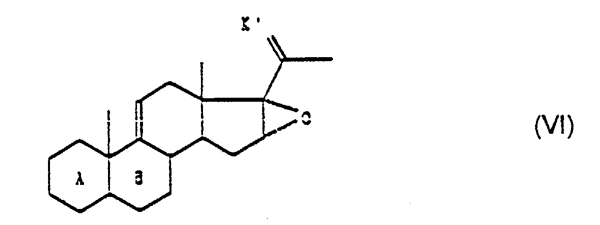 zu erhalten, in welcher K' eine mit K identische oder von K verschiedene Schutzgruppe für die Ketonfunktion der Formel: 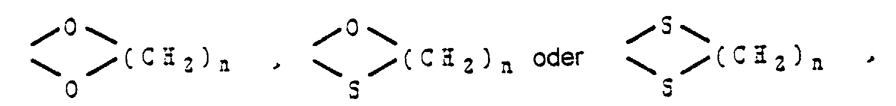 darstellt, wobei n wie vorhergehend definiert ist, die man mit einem organometallischen Methylierungsreagenz behandelt, um das 16β-methyiderivat der Formel (VII): 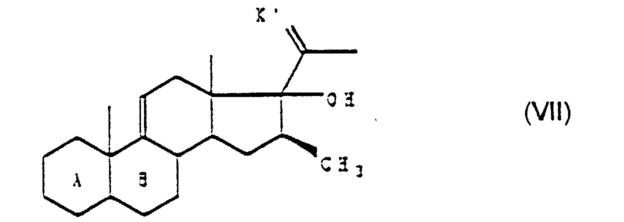 zu erhalten, deren 3- und 20-Keton-Funktionen man freisetzt, um das erwartete Produkt der Formel (I) zu erhalten.

2. Verfahren gemäß Anspruch 1, dadurch gekennzeichnet, daß das Dehydratisierungsmittel Phosphoroxychlorid ist, das in Gegenwart eines tertiären Amins verwendet wird.

3. Verfahren gemäß Anspruch 1, dadurch gekennzeichnet, daß das organometallische Methylierungsreagenz ein Methylmagnesiumhalogenid oder Methyllithium ist.

4. Verfahren gemäß Anspruch 1, dadurch gekennzeichnet, daß das Epoxydierungsreagenz Wasserstoffperoxid ist.

5. Verfahren gemäß Anspruch 1, dadurch gekennzeichnet, daß das organometallische Methylierungsreagenz, das man auf das Epoxid der Formel (VI) wirken läßt, eines der in Anspruch 3 erwähnten ist.

6. Verfahren gemäß einem der Ansprüche 1 bis 5, dadurch gekennzeichnet, daß man zu Beginn eine Verbindung der Formel (II) verwendet, in welcher die Ringe A und B einen Rest: 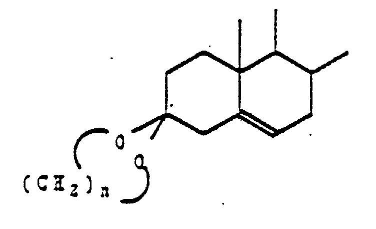 darstellen, wobei n wie vorhergehend definiert und insbesondere gleich 2 ist.

7. Verfahren gemäß einem der Ansprüche 1 bis 6, dadurch gekennzeichnet, daß man die 20-Keto-Funktion durch dasselbe Ketal wie in 3-Position und insbesondere durch eine Ethylendioxygruppe schützt.

8. Als industrielle Produkte die Verbindung der wie in Anspruch 1 definierten Formel (III), in welcher K die wie in Anspruch 1 definierten Werte besitzt, mit Ausnahme des Oxorestes, sowie die Verbindungen der wie in Anspruch 1 definierten Formeln (IV), (V), (Vl) und (VII), unter Ausschluß der Verbindungen 3,3-Ethylendioxy-5,9(11),16-pregnatrien-20-on und 3,3,20,20-Bis(ethylendioxy)-17α-hydroxy-16β-methyl-5,9(11)-pregnadien.
